# EUROPEAN PATENT APPLICATION

(11) **EP 4 209 150 A1**
(43) Date of publication of application: **12.07.2023**
(21) Application number: 21863524.1
(22) Date of filing: 20.08.2021
(51) Int. Cl.: A47C 27/08, A47C 27/10

(54) **SMART MATTRESS**

(30) Priority: 03.09.2020 CN 202010917520; 03.09.2020 CN 202021903958 U
(71) Applicant: Shanghai Backrobo Wellness Co., Ltd., Shanghai 201101 (CN)
(72) Inventor: LIU, Wenchao, Shanghai 201101 (CN); DU, Jichang, Shanghai 201101 (CN)
(74) Representative: Canzler & Bergmeier Patentanwälte Partnerschaft mbB
(86) International application number: PCT/CN2021/113724
(87) International publication number: WO 2022/048457

(57) **Abstract**

A smart mattress, comprising a mattress body (1), and further comprising an airbag assembly (2) disposed on a supporting surface of the mattress body (1). The airbag assembly (2) comprises multiple airbags respectively used to support multiple parts of the human body, and the height of each of the airbags is adjustable. The present smart mattress has a sleep aid function; the air volumes of corresponding airbags in the airbag assembly (2) can be controlled according to a user's experience, so that the heights of the corresponding airbags are flexibly adjusted to achieve the purpose of floating support of different parts of the human body, and the supporting heights of parts of the mattress are alternately and gently changed to achieve the function of relaxing and massage before sleeping. In addition, the smart mattress further has a wake-up function and portability; the mattress body (1) can be folded for carrying with one; moreover, an air pump (41) is configured to be columnar, and after the mattress body (1) is folded, the air pump (41) can be wrapped inside; thus, the smart mattress has excellent portability and can be flexibly applied to various occasions.

## Description

### TECHNICAL FIELD

The present disclosure relates to a mattress, in particular to a smart mattress capable of sleep aid through massage.

### BACKGROUND OF THE INVENTION

Along with a quickening pace of modern life, pressure from work and study is increasing, and insomniacs are also increasing. Difficulty in falling asleep as well as short sleep time at night has become a lingering problem for insomniacs. A mattress is a type of article placed between the human body and a bed to ensure consumers to get healthy and comfortable sleep. Mattresses are made of various materials, which can bring different sleep effects to people.

However, mattresses available on the market now are merely used for sleeping, with no other functions, and how to combine traditional mattresses with modern technology to improve sleep quality will be an important subject.

Traditional mattresses are arranged vertically through independent springs and packed with non-woven fabrics. At the same time, latex, sound insulation cotton and the like are used as mattress filling layers, to make the human body fall asleep in a comfortable state through the softness and hardness of mattresses. In terms of another type of mattress, part of the filling sponge is replaced with an inflatable airbag, to change the hardness of the mattress through different inflatable pressure, in order to achieve a purpose of adjusting the supporting force. The above two types of mattresses can merely provide a single supporting function, and do not realize sleep aid, relaxing, massage and body adjustment for users.

In addition, traditional mattresses are thick and heavy, making them inconvenient to carry. When traveling, participating in outdoor activities, or attending sports and leisure events, it is not possible to use a familiar mattress with sleep aid functions in non-home settings, which can easily lead to poor sleep. This is also a problem that needs to be addressed in the sleep industry.

### SUMMARY OF THE INVENTION

In view of the above shortcomings of the prior art, a technical problem to be solved in the present disclosure is to provide a portable smart mattress which may aid people in sleep through massage and which may monitor the sleep state in real time.

To achieve the above object, the present disclosure provides a smart mattress, including a mattress body. The smart mattress further includes an airbag assembly disposed on a supporting surface of the mattress body, the airbag assembly comprises a plurality of airbags respectively used to support a plurality of parts of the human body, wherein a height of each of the plurality of airbags is adjustable.

Further, the smart mattress further includes an air source assembly to control an air volume of each of the plurality of airbags in the airbag assembly.

Further, the smart mattress further includes an operation module for a user to control the airbag assembly.

Further, the airbag assembly further comprises an air pump, a solenoid valve group and a signal processing module, wherein the air pump is communicated with the plurality of airbags of the airbag assembly via a plurality of solenoid valves of the solenoid valve group, and the operation module is in communication connection with the signal processing module, and controls opening and closing states of the plurality of solenoid valves in the solenoid valve group through the signal processing module.

Further, the smart mattress further includes a heating assembly disposed on part of the plurality of airbags of the airbag assembly, wherein the heating assembly is used to change the body temperature of a user.

Further, the heating assembly is set to be an electrical heating element which is controlled by an operation module.

Further, the smart mattress further includes a sensor to monitor parameters of the heart rate, respiration and body motion of the human body, wherein an air volume of each of the plurality of airbags in the airbag assembly is adjusted based on data measured by the sensor.

Further, at least one of the plurality of airbags in the airbag assembly is a back airbag, or a waist airbag, or a leg airbag or a foot airbag.

Further, the mattress body has a foldable structure, and upon unfolding, the mattress body is a flat mattress structure.

Further, the mattress body is a sponge mattress.

As mentioned above, the smart mattress involved in the present disclosure has the following beneficial effects:
1. Sleep aid function: the air volumes of corresponding airbags in the airbag assembly can be controlled according to a user's experience, so that the heights of the corresponding airbags are flexibly adjusted to achieve the purpose of floating support of different parts of the human body, and the supporting heights of parts of the mattress are alternately and gently changed to achieve the function of relaxing and massage before sleeping;
2. Wake-up function: the heights of corresponding airbags can be adjusted flexibly, to achieve the purpose of floatingly support different parts of the human body, such that the mattress has a wake-up function;
3. Portability function: the mattress body can be folded for portable use, and the air pump is set to be columnar, after the mattress body is folded, the air pump can be wrapped inside, therefore, the mattress has excellent portability, and can be flexibly applied to various occasions;
4. Functions of sleep aid, wake-up, somatosensory alarm clocks and massage can be achieved through a combination of an air pump, a solenoid valve group and an airbag in the airbag assembly;
5. The temperature of the heating assembly can be controlled, and the function of guiding to fall asleep or guiding to wake up is achieved through a change in temperature;
6. The air volume and action sequence of each airbag in the airbag assembly and the temperature of the heating assembly can be controlled according to the measured heart rate, respiration and body motion data; and
7. An effect of floating support can be generated through adjusting the air volume of each airbag in the airbag assembly, to achieve an effect of massage and relaxing.

### BRIEF DESCRIPTION OF THE DRAWINGS

Fig. 1 is a structural schematic diagram of a smart mattress according to Embodiment 1 of the present disclosure.
Fig. 2 is a structural schematic diagram of a smart mattress according to Embodiment 2 of the present disclosure.
Fig. 3 is a structural schematic diagram of a smart mattress in a folded state according to Embodiment 2 of the present disclosure.

### Reference numerals:

| | | | |
|---|---|---|---|
| 1 | mattress body | 3 | air source assembly |
| 2 | airbag assembly | 4 | operation module |
| 21 | back airbag | 41 | air pump |
| 22 | waist airbag | 42 | solenoid valve group |
| 23 | leg airbag | 43 | signal processing module |
| 24 | foot airbag | 5 | heating assembly |
| 25 | head airbag | 51 | heating sheet I |
| 26 | airbag in the center of the back | 52 | heating sheet II |
| 27 | airbag in the center of the waist | 6 | sensor |
| 28 | hip airbag | 7 | storage bag |

### DETAILED DESCRIPTION

Embodiments of the present disclosure are described below from specific embodiments, and those skilled in the art may apparently understand other advantages and beneficial effects of the present disclosure from the contents disclosed in the present specification.

It should be noted that the structures, proportions and sizes shown in the drawings of the present specification are merely used for matching with the contents disclosed in the specification for easy understanding and reading by those skilled in the art, and are not intended to limit the conditions under which the present disclosure can be implemented, therefore, such structures, proportions and sizes have no technical significance. Any modification of the structures, change of proportional relations or adjustment of sizes shall all fall within the scope covered by the technical contents disclosed in the present disclosure without affecting the effects and objects that can be achieved by the present disclosure. Meanwhile, the terms such as "top", "bottom", "left", "right", "center" and "one" used in the present specification are only for convenience of description, and are not intended to limit the scope of the present disclosure, and the change or adjustment of their relative relations shall also be regarded as falling within the scope of the present disclosure without substantial changes in the technical content.

### Embodiment 1

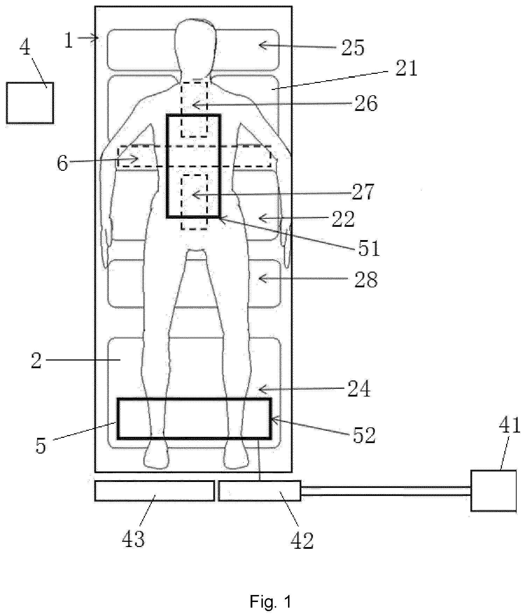

As shown in Fig. 1, the present embodiment provides a smart mattress, including a mattress body 1, the smart mattress further includes an airbag assembly 2 provided on a supporting surface of the mattress body 1, the airbag assembly 2 includes a plurality of airbags respectively used to support multiple parts of the human body, and the heights of the airbags can be adjusted. When the smart mattress is used, through adjusting the heights of the plurality of airbags in the airbag assembly 2 in a certain sequence, the massage function for the human body is realized, thereby further helping the user to fall asleep smoothly or wake up from sleep, to achieve the purpose of massage, relaxing, sleep aid or vitality wake-up. The mattress in the present embodiment is a smart sleep aid mattress, the airbag of the airbag assembly 2 is provided on the supporting surface of the mattress body 1, and the height of the airbag is adjusted through controlling the air volume of the airbag.

As shown in Fig. 1, the smart mattress in the present embodiment further includes a heating assembly 5 arranged on part of the airbags of the airbag assembly 2, and the heating assembly 5 may change the body temperature of the user. The heating temperature of the heating assembly 5 is controllable and changeable. When the heating assembly 5 is used, the heating assembly 5 can change the body temperature of the user to achieve the purpose of guiding to fall asleep or guiding to wake up.

As shown in Fig. 1, the smart mattress in the present embodiment further includes an operation module 4 for the user to control by himself to control the airbag assembly 2 and the heating assembly 5. The operation module 4 can be a remote controller, a wire controller or an intelligent terminal that controls the airbag assembly 2 and the heating assembly 5 through network signals. Specifically, the airbag assembly 2 in the present embodiment further includes an air pump 41, a solenoid valve group 42 and a signal processing module 43. The air pump 41 is respectively communicated with several airbags of the airbag assembly 2 via several solenoid valves of the solenoid valve group 42, the operation module 4 is in communication connection with the signal processing module 43, and controls the opening or closing state of the several solenoid valves in the solenoid valve group 42 through the signal processing module 43. The communication connection mentioned in the present embodiment can be wifi, bluetooth, 5G network, wired transmission and the like; the several airbags of the airbag assembly 2 are adapted to the curved structure of human body surface, to ensure comfort when lying down. In addition, the mattress body 1 in the present embodiment is set to be a sponge mattress.

To optimize configuration, the airbag assembly 2 includes but is not limited to the following airbags:
A head airbag 25 supporting head, the head airbag 25 is like a pillow, and its height can be adjusted manually or automatically according to set pressure;
A back airbag 21 supporting the back of the human body;
A mid-back airbag 26 supporting center of back of human body;
A waist airbag 22 supporting the waist of the human body;
A mid-waist airbag 27 supporting center of the waist of the human body;
A hip airbag 28 supporting hips of the human body;
A foot airbag 24 supporting feet and lower legs.

The above airbags can be arranged according to functional requirements, and the user may fine-tune specific positions of each of the above airbags according to requirements. The air volume of the above air bag can be adjusted automatically or manually.

In the present embodiment, the heating assembly 5 is set to be an electrical heating element which can be controlled by the operation module 4. The heating assembly 5 includes a first heating sheet 51 and a second heating sheet 52 which are controlled by the operation module 4. The first heating sheet 51 and the second heating sheet 52 are both electrical heating sheets. The first heating sheet 51 is attached to the back airbag 21 and the waist airbag 22, to fit most area of the back of the human body, and the second heating sheet 52 is attached to the foot airbag 24, to fit lower legs and feet, so as to achieve an effect of heating while improving comfort. The structural form and quantity of the heating assembly 5 are not limited to the solution disclosed in the present embodiment, and can be customized according to customer requirements.

As shown in Fig. 1, the smart mattress in the present embodiment further includes a sensor 6 to monitor parameters such as the heart rate, respiration and body motion of the human body, and the temperature of the heating assembly 5, and the air volume and action state of each airbag in the airbag assembly 2 are adjusted and set based on data measured by the sensor 6. In the present embodiment, in order to better monitor the parameters of the heart rate, respiration and body motion of the human body, the sensor 6 is placed on the mattress body 1, and fit the human body surface in a supporting manner, to facilitate to collect the parameters of the heart rate, respiration and body motion of the human body, and in the present embodiment, related parameters detected by the sensor 6 include but are not limited to parameters of the heart rate, respiration and body motion of the human body.

In the present embodiment, when a user uses the smart mattress, the sensor 6 is placed on the mattress body 1 to collect parameters of the heart rate, respiration and body motion of the human body. After receiving the parameters of heart rate, respiration and body motion of the human body, the operation module 4 controls the air pump 41, the solenoid valve group 42, the first heating sheet 51 and/or the second heating sheet 52 to execute actions through algorithm processing; through the opening and closing actions of the solenoid valves in the solenoid valve group 42, the air volume of each airbag in the airbag assembly 2 changes floatingly, such as inflation and exhaust actions, so as to adjust the supporting height of each airbag in the airbag assembly 2, so as to produce high and low changes, drag the human body to move slightly in a vertical direction or in a horizontal direction, to form slow massage, and further to eliminate local muscle tension. The function of waking up the human body can also be achieved through this change in the vertical direction, just like a "somatosensory alarm clock". At the same time, the temperature of the first heating sheet 51 and/or the second heating sheet 52 can be selected to be controlled, to improve the user's experience.

The smart mattress in the present embodiment has the following functions:
1. Sleep aid function: the sensor 6 is used to detect the parameters of the heart rate, respiration and body motion of the user, and according to the parameters, massage actions are generated and recommended to the user or the user selects his favorite sleep aid program by himself. The sleep aid program includes but is not limited to the following embodiments: (1) heights of parts of the mattress are alternately and gently changed to simulate the action of a cradle; (2) a relaxing action of periodically lifting the height of limbs to make blood flow back to the body; and (3) the change of temperature; according to user requirements, music players can be added as considered in the later stage to enhance the sleep aid effect.
2. Wake-up function: the sleep state, heart rate, respiration and body motion of the user are obtained according to the data obtained by the sensor 6, a wake-up program is generated according to the user data, or the program preselected by the user is implemented to implement a wake-up action. The wake-up action includes but is not limited to: (1) a stretching action of raising the height of the central position of the back; (2) an action of raising the local height of the shoulders and the back; (3) an action of crossed raising and stretching of the shoulders and the waist; and (4) the change of temperature.
3. Somatosensory alarm clock function: the sleep state of the user is tracked in real time according to the data acquired by the sensor 6, and a wake-up program is implemented at a light sleep period nearest to the time set by the user or at the time specified by the user.

As to the smart mattress in the present embodiment, massage and heating up can be adopted to aid the user in sleep and wake up the user, the sleep state can be monitored in real time, and the mode of sleep aid and wake-up can be automatically selected according to the monitored results and the setting of the user, so as to achieve an effect that the bed moves along with people.

### Embodiment 2

The smart mattress provided in the present embodiment is a portable massage and sleep aid mattress, which can make the body of the user fit more closely to the mattress. The mattress can use massage to aid in sleep, therefore, the mattress is more adjustable compared with traditional mattresses, more economical and comfortable than an electric bed, and has low noise generated in the using process. At the same time, the mattress is portable and may make the bed move along with people.

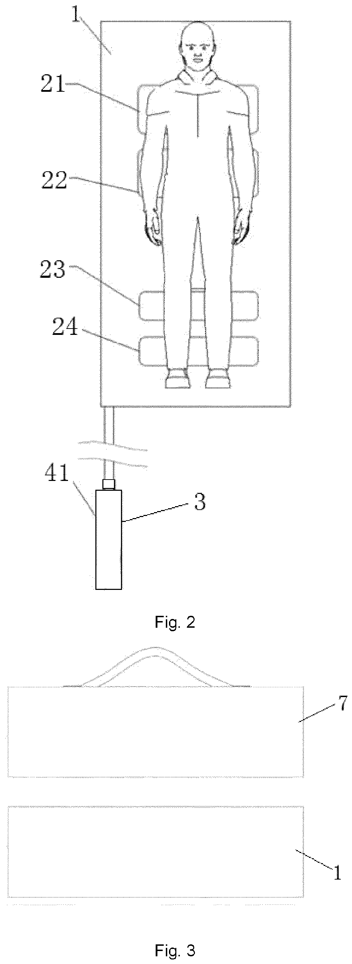

As shown in Fig. 2, the smart mattress in the present embodiment includes a mattress body 1, and further includes an airbag assembly 2 arranged on the supporting surface of the mattress body 1.

The above mattress body 1 is set in a foldable structure and is convenient for users to carry, and can be unfolded into a flat mattress structure. In the present embodiment, the mattress body 1 is set as a light and handy portable mattress structure which is convenient to lift with one hand. The mattress body 1 adopts a multi-layer structure and can be freely curled and put into a storage bag 7 as shown in Fig. 3.

The airbag assembly 2 in the present embodiment includes several airbags with controllable air volume and inflation sequence, and may support the back, waist, legs and feet of the human body in varying actions, and through various changing actions of the supporting positions of the airbags, the purpose of sleep aid, wake-up, relaxing and massage is achieved. The setting of heights of the several airbags of the airbag assembly 2 is suitably matched with the normal physiological curve structure of the human body, and is freely adjusted and set according to user requirements. A heating assembly 5 can be arranged on the airbag of the airbag assembly 2 to improve the user's experience.

The airbag assembly 2 in the present embodiment at least includes:
A back airbag 21 that supports the back of the human body, where the width of the back airbag 21 is greater than the width of the back of the human body, to achieve an effect of complete fit and support, and ensure comfortness when the user lies on the back;
A waist airbag 22 that supports the waist of the human body, where the width of the waist airbag 22 is greater than the width of the waist of the human body, to achieve an effect of complete fit and support, and ensure comfortness when the user lies on the back;
A leg airbag 23 that supports legs of the human body, where the width of the leg airbag 23 is greater than the width of legs of the human body, to achieve an effect of complete fit and support, and ensure comfortness when the user lies down;
A foot airbag 24 that supports feet of the human body, where the width of the foot airbag 24 is greater than the width of feet of the human body, to achieve an effect of complete fit and support, and ensure comfort when the user lies down.

The smart mattress in the present embodiment further includes an air source assembly 3, the air source assembly 3 may control the air volumes of all the airbags in the airbag assembly 2, and the supporting heights of all the airbags of the airbag assembly 2 can be adjusted and set under the control of the air source assembly 3.

As a preferred solution, the air source assembly 3 is set to a columnar air pump 41, air passages of the air pump 41 are divided into several passages and are respectively communicated with the several airbags of the air bag assembly 2, and solenoid valves are arranged on several air passages of the air pump 41 to control the volume of inflated air.

To enhance the portability function, after curling on the mattress body 1, the columnar air pump 41 is wrapped by the mattress body 1, and can be arbitrarily carried to multiple occasions.

In the present embodiment, a control panel is arranged for the air pump 41, the control panel is an operation module 4, the user may lie on the mattress, and manually manipulate the control panel, to freely manipulate the inflation process of the air pump 41 according to the feeling of his body, that is, the opening sequence of the solenoid valves and the inflation speed of the air pump 41, or to select different automatic programs, to further control the volume of inflated air and inflation sequence of airbags of the airbag assembly 2, such that the height of each airbag of the airbag assembly 2 is changed, and through a change of the airbag in a vertical direction, the human body is dragged to move slightly in the vertical direction and a horizontal direction, and local muscle tension can be eliminated through slow massage.

Under the manipulation of the air pump 41, at least the following functions can be achieved:
Sleep aid function: the user can select a favorite sleep aid mode. The sleep aid mode includes but is not limited to the following embodiments: 1. the heights of parts of the mattress are alternately and gently changed to achieve the function of relaxing and massage before sleeping; 2. the height of lower limbs is raised appropriately, to make blood flow back to the heart and enhance comfort before sleeping.

Wake-up function: the wake-up actions include: 1. the heights of parts of the mattress are alternately and gently changed to achieve the function of wake-up and massage during sleep; 2. crossed raising and stretching actions of the shoulders and the waist.

In the present embodiment, the airbag is applied to the mattress to support various parts of the human body with air and simultaneously drive the movement of the body to achieve the purpose of soothing and relaxing and help the user to fall asleep quickly.

The above are preferred embodiments of the present disclosure, based on which those skilled in the art may make various modifications or improvements, such as changing all or part of the medium in the airbag from air to liquid, and these modifications or improvements shall all fall within the protection scope of the present disclosure without departing from the general concept of the present disclosure.

## Claims

1. A smart mattress, comprising a mattress body (1), wherein the smart mattress further comprises an airbag assembly (2) disposed on a supporting surface of the mattress body (1), wherein the airbag assembly (2) comprises a plurality of airbags respectively used to support a plurality of parts of the human body, wherein a height of each of the plurality of airbags is adjustable.

2. The smart mattress of claim 1, further comprising an air source assembly (3) to control an air volume of each of the plurality of airbags in the airbag assembly (2).

3. The smart mattress of claim 1, further comprising an operation module (4) for a user to control the airbag assembly (2).

4. The smart mattress of claim 3, wherein the airbag assembly (2) further comprises an air pump (41), a solenoid valve group (42) and a signal processing module (43), wherein the air pump (41) is communicated with the plurality of airbags of the airbag assembly (2) via a plurality of solenoid valves of the solenoid valve group (42), and the operation module (4) is in communication connection with the signal processing module (43), and controls opening and closing states of the plurality of solenoid valves in the solenoid valve group (42) through the signal processing module (43).

5. The smart mattress of claim 1, further comprising a heating assembly (5) disposed on part of the plurality of airbags of the airbag assembly (2), wherein the heating assembly (5) is used to change the body temperature of a user.

6. The smart mattress of claim 5, wherein the heating assembly (5) is set to be an electrical heating element which is controlled by an operation module (4).

7. The smart mattress of claim 1, further comprising a sensor (6) configured to monitor parameters of the heart rate, respiration and body motion of the human body, wherein an air volume of each of the plurality of airbags in the airbag assembly (2) is adjusted based on data measured by the sensor (6).

8. The smart mattress of claim 1, wherein at least one of the plurality of airbags in the airbag assembly (2) is a back airbag (21), or a waist airbag (22), or a leg airbag (23) or a foot airbag (24).

9. The smart mattress of claim 1, wherein the mattress body (1) has a foldable structure, and upon unfolding, the mattress body (1) is a flat mattress structure.

10. The smart mattress of claim 1 or 9, wherein the mattress body (1) is a sponge mattress.
